# EUROPEAN PATENT APPLICATION

(11) **EP 3 333 268 A1**
(43) Date of publication of application: **13.06.2018**
(21) Application number: 16290229.0
(22) Date of filing: 09.12.2016
(51) Int. Cl.: C12Q 1/68, G01N 33/574

(54) **BIOMARKER PANEL FOR PROGNOSIS OF BLADDER CANCER**

(71) Applicant: Institut Paoli Calmettes, 13009 Marseille (FR)
(72) Inventor: PIGNOT, Géraldine, 13008 Marseille (FR); BIÈCHE, Ivan, 92150 Suresnes (FR); DAMOTTE, Diane, 75006 Paris (FR); LE GOUX, Constance, 75017 Paris (FR)
(74) Representative: Forstmeyer, Dietmar

(57) **Abstract**

The invention provides a method of determining a prognosis of survival in a bladder cancer patient using messenger RNA (mRNA) or protein expression profiles of specific biomarkers that are present in a tumor sample of said bladder cancer patient. The invention further comprises a kit for determining a non-muscle invasive bladder cancer (NMIBC) or muscle invasive bladder cancer (MIBC) patient's prognosis of recurrence-free or overall survival based upon the mRNA or protein expression profile according to the invention, and also relates to the use of said mRNA or protein expression profile in the prognosis of recurrence-free or overall survival.

## Description

### Field of the Invention

The invention provides a method of determining a prognosis of survival in a bladder cancer patient.

### Background of the Invention

Bladder cancer is the sixth most common cause of cancer mortality and its incidence has increased markedly in recent decades. Urothelial carcinoma is the predominant histologic type in the United States and Europe, accounting for 90% of all cases. About two-thirds of newly diagnosed cases are non-muscle invasive bladder cancer (NMIBC). These have a 60% recurrence rate and, in 10% of cases, evolve to muscle-invasive tumors. Muscle invasive bladder cancer (MIBC) occurs in one-third of cases at diagnosis. There is a dramatic difference in survival rates between early and advanced bladder cancer.

It is, therefore, an aim of the present invention to provide a novel alternative method of determining a prognosis of recurrence-free or overall survival in a bladder cancer patient afflicted with NMIBC or MIBC. An earlier and/or more precise prognosis of recurrence-free or overall survival of the bladder cancer patient would allow the medical practitioner to adjust treatment regimens more effectively, and ultimately to personalize tumor treatment in order to maximize outcome.

### Summary and Description of the Invention

The present invention was made in view of the prior art and the needs described above, and, therefore, the object of the present invention is to provide a novel alternative method of determining a prognosis of recurrence-free survival in a NMIBC patient, or a prognosis of recurrence-free or overall survival in a MIBC patient.

More specifically, the inventors found that certain biomarkers are differentially expressed in tumor samples of patients afflicted with NMIBC or MIBC as compared to relevant controls.

The inventors showed that OX40L overexpression is significantly associated with a higher risk of recurrence in NMIBC and MIBC.

The inventors further showed that a certain FOXP3/CD8 ratio is indicative for a higher risk of recurrence in NMIBC.

The inventors also showed that the expression level of CD8 and TIGIT is significantly associated with prognosis in terms of recurrence-free and overall survival in MIBC.

The inventors, moreover, showed that OX40L, CD8 and TIGIT are differentially expressed in MIBC patients thereby allowing to differentiate between MIBC patients which are at high risk for relapse or in need of chemotherapy or other treatment to improve their chances of survival and those with good prognosis, i.e. recurrence-free survival and/or likely to experience a better overall survival.

All in all, the findings of the invention allow to identify those NMIBC and MIBC patients who are likely to experience a recurrence and/or metastasis of their bladder cancer after treatment of the primary tumor by transurethral bladder resection or radical cystectomy as well as those MIBC patients that have a poor overall survival prognosis.

The objects of the present invention are solved by the subject matter of the attached claims.

The aspects of the present invention will become apparent upon reference to the following detailed description.

The present invention provides a method of determining a prognosis of a recurrence-free survival in a bladder cancer patient comprising:
determining messenger ribonucleic acid (mRNA) or protein expression levels for a gene panel in a tumor sample of a bladder cancer patient;
wherein the gene panel consists of CD8 (NM_001768.6, CD8a molecule), FOXP3 (NM_014009.3, forkhead box P3), TIGIT (NM_173799.3, T cell immunoreceptor with Ig and ITIM domains) and OX40L (NM_003326.4, tumor necrosis factor (ligand) superfamily, member 4);
wherein a bladder cancer patient having a high expression level for OX40L, and (i) a normal expression level for each of CD8 and TIGIT or (ii) a FOXP3/CD8 ratio of≥ 6.25 (i.e. equal or more than 6.25), has a prognosis of a decreased probability of recurrence-free survival.

The gene and protein expression profiles of the present invention consists of a group of genes and proteins that are differentially expressed (e.g., up-regulated or down-regulated) in patients whose bladder cancer is likely to recur after treatment of the primary tumor. Specifically, some of these genes and their encoded proteins are up-regulated (over-expressed) in bladder cancer patients whose cancers are likely to recur/metastasize after treatment of the primary tumor, relative to expression of the same genes in normal bladder tissue of said patients or the primary bladder cancer tumors of patients whose cancers are unlikely to recur/metastasize. The gene and protein expression profiles of the present invention thus can be used to predict the likelihood of recurrence of the cancer and/or disease-related death. The present gene and protein expression profiles also can be used to identify those bladder cancer patients requiring adjuvant therapies.

The gene and protein expression profiles of the present invention provides the clinician with a prognostic tool capable of providing valuable information that can positively affect management of the disease. Oncologists can assay the primary tumor for the presence of the biomarkers (genes and proteins) of the invention, and thus identify with a high degree of accuracy those patients whose disease is likely to recur or metastasize. This information, taken together with other available clinical information, allows more effective management of the disease.

Table 1 identifies the genes of the gene panel of the present invention. Table 1 includes the NCBI Accession No. of a variant of each gene; other variants of these genes can exist, which can be readily ascertained by reference to an appropriate database such as NCBI Entrez (available via the NIH website). Alternate names for the genes listed in Table 1, in addition to those indicated, also can be determined from the NCBI site.

**Table 1: Genes of the gene panel**

| Gene (alternate name) | Accession No. | Gene Definition |
|---|---|---|
| CD8 (CD8A) | NM_001768.6 | CD8a molecule |
| FOXP3 | NM_014009.3 | forkhead box P3 |
| OX40L (TNFSF4) | NM_003326.4 | tumor necrosis factor (ligand) superfamily, member 4 |
| TIGIT (VSIG9 ; VSTM3) | NM_173799.3 | T cell immunoreceptor with Ig and ITIM domains |

Table 2 identifies the proteins encoded by the genes of the gene panel of the present invention. Table 2 includes the NCBI Accession No. of a variant of each protein; other variants of these proteins may exist, which can be readily ascertained by reference to an appropriate database such as NCBI Entrez (available via the NIH website). Alternate names for the proteins listed in Table 2 also can be determined from the NCBI site.

**Table 2: Proteins encoded by the genes of the gene panel**

| Gene | Encoded Protein | Accession No. |
|---|---|---|
| CD8 | 235 aa protein (T-cell surface glycoprotein CD8 alpha chain isoform 1 precursor) | NP_001139345.1 |
| FOXP3 | 465 aa protein (forkhead box protein D1) | NP_004463.1 |
| OX40L | 183 aa protein (tumor necrosis factor ligand superfamily member 4 isoform 1) | NP_003317.1 |
| TIGIT | 244 aa protein (T-cell immunoreceptor with Ig and ITIM domains precursor) | NP_776160.2 |

The practice of the present invention will employ, unless otherwise indicated, conventional techniques of molecular biology (including recombinant techniques), microbiology, cell biology, and biochemistry, which are within the skill of the art.

Unless defined otherwise, technical and scientific terms used herein have the same meaning as commonly understood by one of ordinary skill in the art to which this invention belongs. For convenience, the meaning of certain terms and phrases employed in the specification, examples, and appended claims are provided below. The definitions are not meant to be limiting in nature and merely serve to provide a clearer understanding of certain aspects of the present invention.

The term "gene" refers to a nucleic acid sequence that comprises control and coding sequences necessary for producing a polypeptide or precursor. A gene may contain one or more modifications in either the coding or the untranslated regions that could affect the biological activity or the chemical structure of the expression product, the rate of expression, or the manner of expression control. Such modifications include, but are not limited to, mutations, insertions, deletions, and substitutions of one or more nucleotides. The gene may constitute an uninterrupted coding sequence or it may include one or more introns, bound by the appropriate splice junctions. The term "gene" as used herein includes variants of the genes identified in Table 1.

The term "gene expression" refers to the process by which a nucleic acid sequence undergoes successful transcription and translation such that detectable levels of the nucleotide sequence are expressed.

The term "gene expression profile" refers to a group of genes expressed by a particular cell or tissue type wherein presence of the genes taken together or the differential expression of such genes, is indicative/predictive of a certain condition.

The terms "differentially expressed gene", "differential gene expression", and their synonyms, which are used interchangeably, refer to a gene whose expression is activated to a higher or lower level in a subject suffering from a disease, specifically cancer, such as bladder cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about three-fold, preferably at least about four-fold, more preferably at least about six-fold, most preferably at least about ten-fold difference between the expression of a given gene in normal and diseased subjects, or between various stages of disease development in a diseased subject.

The term "over-expression" with regard to an RNA transcript is used to refer to the level of the transcript determined by normalization to the level of reference mRNAs, which might be all measured transcripts in the specimen or a particular reference set of mRNAs. For the purpose of this invention, "over-expression" is considered to be present when there is at least an about three-fold difference between the mRNA value of the target gene relative to a standard (e.g. a reference gene).

The term "under-expression", "low expression", "relatively low expression", or "lower expression level(s)" and synonyms thereof with regard to an RNA transcript are used to refer to the level of the transcript determined by normalization to the level of reference mRNAs, which might be all measured transcripts in the specimen or a particular reference set of mRNAs. For the purpose of this invention, "under-expression" is considered to be present when there is at least an about three-fold difference between the mRNA value of the target gene relative to a standard (e.g. a reference gene).

With respect to proteins encoded by genes, the term "differential expression" refers to both quantitative as well as qualitative differences in the temporal and tissue expression patterns of a protein in a diseased subject (or diseased tissue or cells) versus a normal or control subject (or normal adjacent tissue or cells). For example, a differentially expressed protein may be up-regulated (over-expressed) or down-regulated (under-expressed) in a disease condition versus a normal condition. Stated another way, a protein is differentially expressed when expression of the protein occurs at a higher or lower level in the diseased tissues or cells of a patient relative to the level of its expression in the normal (disease-free) tissues or cells of the patient and/or control tissues or cells.

The term "prognosis" is used herein to refer to the prediction of the likelihood / probability of cancer-attributable death or progression, including recurrence and metastatic spread.

The term "prediction" is used herein to refer to the likelihood/probability that a patient will survive, following surgical removal of the primary tumor and/or chemotherapy for a certain period of time without cancer recurrence. The predictive method of the present invention can be a valuable tool in predicting whether overall (or long-term) survival of the patient, following surgery and/or termination of chemotherapy or other treatment modalities is likely.

The term "overall survival" is used herein to refer to survival for at least 2 years, at least 5 years, at least 8 years, or at least 10 years, following surgery or other treatment.

The term "tumor" as used herein, refers to all neoplastic cell growth and proliferation, whether malignant or benign, and all pre-cancerous and cancerous cells and tissues.

The term "cancer" refers to or describes the physiological condition in mammals that is typically characterized by unregulated cell growth.

The term "nucleic acid" as used herein, refers to a molecule comprised of one or more nucleotides, i.e., ribonucleotides, deoxyribonucleotides, or both. The term includes monomers and polymers of ribonucleotides and deoxyribonucleotides, with the ribonucleotides and/or deoxyribonucleotides being bound together, in the case of the polymers, via 5' to 3' linkages. The ribonucleotide and deoxyribonucleotide polymers may be single or double-stranded. However, linkages may include any of the linkages known in the art including, for example, nucleic acids comprising 5' to 3' linkages. The nucleotides may be naturally occurring or may be synthetically produced analogs that are capable of forming base-pair relationships with naturally occurring base pairs. Examples of non-naturally occurring bases that are capable of forming base-pairing relationships include, but are not limited to, aza and deaza pyrimidine analogs, aza and deaza purine analogs, and other heterocyclic base analogs, wherein one or more of the carbon and nitrogen atoms of the pyrimidine rings have been substituted by heteroatoms, e.g., oxygen, sulfur, selenium, phosphorus, and the like. Furthermore, the term "nucleic acid sequences" contemplates the complementary sequence and specifically includes any nucleic acid sequence that is substantially homologous to the both the nucleic acid sequence and its complement.

The terms "array" and "microarray" refer to the type of genes or proteins represented on an array by oligonucleotides or protein-capture agents, and where the type of genes or proteins represented on the array is dependent on the intended purpose of the array (e.g., to monitor expression of human genes or proteins). The oligonucleotides or protein-capture agents on a given array may correspond to the same type, category, or group of genes or proteins. Genes or proteins may be considered to be of the same type if they share some common characteristics such as species of origin (e.g., human, mouse, rat); disease state (e.g., cancer); functions (e.g., protein kinases, tumor suppressors); or same biological process (e.g., apoptosis, signal transduction, cell cycle regulation, proliferation, differentiation). For example, one array type may be a "cancer array" in which each of the array oligonucleotides or protein-capture agents correspond to a gene or protein associated with a cancer.

As used herein "determining messenger ribonucleic acid (mRNA) or protein expression levels in a tumor sample" means assaying a test sample, e.g. a paraffin-embedded biopsy sample from a patient, *in vitro* to determine the concentration or amount of the mRNAs or proteins in the sample. Any convenient qualitative, semi-quantitative or, preferably, quantitative detection method for determining nucleic acids or proteins can be used to determine the concentration or amount of the mRNAs or proteins in the sample. A variety of methods for determining mRNA, e.g. determination by nucleic acid hybridization and/or nucleic acid amplification, and proteins, e.g. determination by protein staining, are well known to those of skill in the art. Exemplary methods to determine the concentration or amount of the mRNAs or proteins in a sample are provided below.

The concentration or amount of the mRNA or protein in the sample may be directly determined in the test sample, that is, without a RNA or protein extraction step. Alternatively, RNA or protein may be extracted from the test sample prior to processing for detection. RNA may be purified using a variety of standard procedures as described, for example, in RNA Methodologies, A laboratory guide for isolation and characterization, 2nd edition, 1998, Robert E. Farrell, Jr., Ed., Academic Press. In addition, there are various processes as well as products commercially available for isolation of RNAs. For example, RNA may be isolated by organic extraction followed by purification on a glass fiber filter. Proteins may be purified using a variety of standard procedures as described, for example, in Basic Methods in Protein Purification and Analysis: A Laboratory Manual, 2011, Richard J. Simpson, Ed., CSH Press (ISBN 978-087969-867-6). For example, a protein may be isolated by extraction and chromatographic purification.

The method is based on comparing the patient's expression levels of the mRNAs or proteins in the sample with those obtained using relevant controls, e.g. internal standards, tissue samples from subjects known to be free of the disease or known to suffer from the same or a different disease. In cases where the method is being used to monitor a patient or to test for the recurrence or progression of the disease, the "control" may be test results obtained from the same patient at an earlier time, i.e., the patient may be examined for changes in mRNA levels before and after a certain treatment.

It will be understood that it is not absolutely essential that an actual control sample be run at the same time that assays are being performed on a test sample. Once "normal", i.e. control levels of the mRNAs or proteins (or of mRNA or protein ratios) have been established, these levels can provide a basis for comparison without the need to rerun a new control sample with each assay. The comparison between the test and control samples provides a basis for a conclusion as to whether a subject has a good or poor prognosis for recurrence-free or overall survival. The expression levels of the analyzed mRNAs or proteins when statistically analyzed will have a threshold whereby expression levels of the individual mRNAs above or below the threshold are indicative for the good or poor prognosis. Threshold values for each of the analyzed biomarkers of the gene panel of the invention can be determined by any suitable algorithm. Such an algorithm may involve classifying a sample between recurrence-free and recurrence groups. For example, samples may be classified on the basis of threshold values as indicated, or based upon Mean and/or Median mRNA or protein levels in bladder cancer patients versus a non-bladder cancer population (e.g., a cohort from the general population or a patient cohort with diseases other than bladder cancer). Various classification schemes are known for classifying samples between two or more groups, including Decision Trees, Logistic Regression, Principal Components Analysis, Naive Bayes model, Support Vector Machine model, and Nearest Neighbor model. In addition, the predictions from multiple models can be combined to generate an overall prediction.

The mRNA or protein expression profile can be generated from the samples using any of various methods known in the art for quantifying mRNA or protein levels. Methods for quantifying mRNA include polymerase-based assays, such as Real-Time quantitative PCR (e.g., Taqman™), hybridization-based assays, for example using microarrays (e.g. Affymetrix U133 GeneChip), nucleic acid sequence based amplification (NASBA), flap endonuclease-based assays, as well as direct RNA capture with branched DNA (QuantiGene™), Hybrid Capture™ (Digene), or nCounter™ mRNA detection (nanostring). The assay format, in addition to determining the mRNA levels will also allow for the control of, inter alia, intrinsic signal intensity variation. Such controls may include, for example, controls for background signal intensity and/or sample processing, and/or hybridization efficiency, as well as other desirable controls for quantifying mRNA levels across samples (e.g., collectively referred to as "controls"). Many of the assay formats for amplifying and quantitating mRNA sequences, and thus for generating mRNA profiles are commercially available. Methods for determining gene and/or protein expression in sample tissues are described, for example, in U.S. Pat. No. 6,271,002; U.S. Pat. No. 6,218,122; U.S. Pat. No. 6,218,114; and U.S. Pat. No. 6,004,755, or references cited therein.

Although the mRNAs tested for are indicated as RNA sequences, it will be understood that, when referring to hybridizations or other assays, corresponding DNA sequences can be used as well. For example, RNA sequences may be reverse transcribed and amplified using the polymerase chain reaction (PCR) in order to facilitate detection. In these cases, it will actually be DNA and not RNA that is directly quantitated. It will also be understood that the complement of the reverse transcribed DNA sequences can be analyzed instead of the sequence itself. In this context, the term "complement" refers to an oligonucleotide that has an exactly complementary sequence, i.e. for each adenine there is a thymine, etc. Although assays may be performed for the mRNAs individually, it is generally preferable to assay several mRNAs or to compare the ratio of two or more of the mRNAs.

In a preferred embodiment of the method of the invention, the bladder cancer patient is a muscle invasive bladder cancer (MIBC) patient.

In another preferred embodiment of the method of the invention, the bladder cancer patient is a non-muscle invasive bladder cancer (NMIBC) patient.

According to the invention, the tumor sample can be a paraffin-embedded biopsy sample.

The method of the invention can comprise determining a prognosis of a recurrence-free survival in a NMIBC patient comprising:
determining mRNA or protein expression levels for a gene panel in a tumor sample of the patient; wherein the gene panel consists of CD8, FOXP3 and OX40L;
wherein a NMIBC patient having a high expression level for OX40L, and a FOXP3/CD8 ratio of≥ 6.25 has a prognosis of a decreased probability of recurrence-free survival, i.e. a higher risk of recurrence.

The method of the invention can also comprise determining a prognosis of a recurrence-free survival in a MIBC patient comprising:
determining mRNA or protein expression levels for a gene panel in a tumor sample of the patient; wherein the gene panel consists of CD8, TIGIT and OX40L;
wherein a MIBC patient having a high expression level for OX40L and a normal expression level for each of CD8 and TIGIT has a prognosis of a decreased probability of recurrence-free survival, i.e. a higher risk of recurrence.

The method of the invention can further comprise determining a prognosis of overall survival in a MIBC patient, wherein the method comprises:
determining mRNA or protein expression levels for a gene panel in a tumor sample of the patient; wherein the gene panel consists of CD8, TIGIT and OX40L;
wherein a MIBC patient having a high expression level for OX40L and a normal expression level for each of CD8 and TIGIT has a prognosis of a decreased probability of overall survival, i.e. a relatively lower chance of survival and thus a higher risk of death.

Preferably, the method of the invention can comprise determining the mRNA expression profile of the genes of the gene panel by an amplification- and/or hybridization-based assay. Currently preferred amplification- and/or hybridization-based assays include quantitative real-time polymerase chain reaction (RT-PCR) or mRNA microarray analysis.

Preferably, the method of the invention can comprise determining the protein expression profile of the proteins encoded by the genes of the gene panel by immunohistochemistry.

The method of the invention further may comprise determining the level of one or more normalization control(s) in the sample. The control can include reference or control genes and the proteins expressed thereby. Reference or control genes are genes that are consistently expressed in many tissue types, including cancer and normal tissues, and thus are useful to normalize gene or protein expression profiles. According to a preferred embodiment of the invention, the control gene is an endogenous RNA control gene. The currently preferred control gene is TBP (Accession No. NM_003194) which encodes the TATA box-binding protein. In an alternative preferred embodiment of the invention, the normalization control can be a non-endogenous RNA, or a RNA not expressed in the sample. For example, the normalization control may be one or more exogenously added RNA(s) that are not naturally present in the patient, e.g. an RNA from an other organism, e.g. E. coli, C. elegans or Arabidopsis, and/or one or more human RNAs not expressed in the tumor sample undergoing analysis.

The mRNA or protein expression profile may be prepared with the use of a custom kit or array, e.g., to allow particularly for the profiling of the genes of the gene panel or the proteins encoded thereby. Accordingly, the present invention further provides a kit (or test) for determining a patient's prognosis of recurrence-free or overall survival based upon the biomarkers as described herein.

The kit for determining a MIBC or NMIBC patient's prognosis of recurrence-free or overall survival comprises at least means for determining the expression profile of CD8, FOXP3 and OX40L; or CD8, OX40L and TIGIT; or CD8, FOXP3, OX40L, and TIGIT; in a tumor sample from a patient. The means for determining the expression profile of CD8, OX40L, and FOXP3and/or TIGIT can be oligonucleotide probes specific for CD8, OX40L, and FOXP3and/or TIGIT; or mRNA-specific primers for reverse transcribing or amplifying each of CD8, OX40L, and FOXP3and/or TIGIT. For example, the means for determining the expression profile of CD8, OX40L, and FOXP3and/or TIGIT may be TaqMan probes specific for each mRNA of the gene panel of the invention.

The design of oligonucleotide probes specific for CD8, OX40L, and FOXP3and/or TIGIT; or mRNA-specific primers for reverse transcribing or amplifying each of CD8, OX40L, and FOXP3and/or TIGIT to detect their expression levels in accordance with suitable assay formats is well known to those of skill in the art, and appropriate probes and/or primers can be commercially purchased.

Further, the kit may comprise an enzyme for cDNA preparation (e.g., reverse transcriptase) and/or PCR amplification (e.g., Taq polymerase), and/or a reagent for detecting and/or quantifying mRNA. Additionally, the kit may further include a reagent for mRNA isolation from samples. The kit can also comprise one or more normalization control(s). The normalization control(s) can, for example, be provided as one or more separate reagent(s) for spiking samples or reactions. Preferably, the normalization control(s) is/are selected from endogenous RNA expressed in the sample, e.g. TBP.

The invention further provides the use of the expression profile of the biomarkers of the invention in a tumor sample from a NMIBC or MIBC patient for determining a MIBC or NMIBC patient's prognosis of recurrence-free or overall survival. The mRNA or protein expression profile that is used for determining a MIBC or NMIBC patient's prognosis of recurrence-free or overall survival may further comprise the expression profile of TBP. The mRNA or protein expression profile is indicative of a poor prognosis, i.e. higher risk of recurrence and/or a relatively lower chance of survival, if OX40L is up-regulated (over-expressed), and (i) each of CD8 and TIGIT is normally expressed or (ii) the expression ratio of FOXP3/CD8 is ≥ 6.25 in the tumor sample.

### Description of the Figures

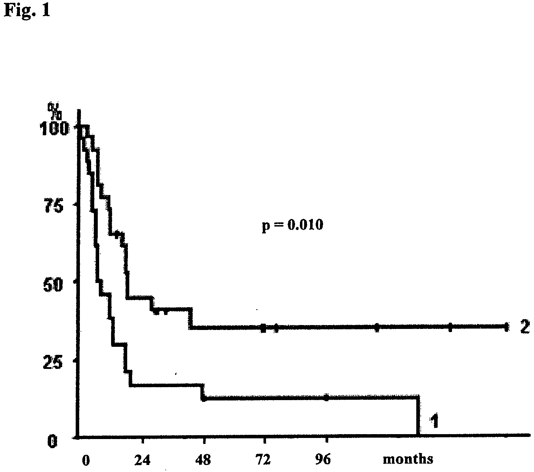

**Figure 1****.** Recurrence-free survival according to *FOXP3*/*CD8* ratio in NMIBC patients. (1) *FOXP3*/*CD8* ratio ≥ 6.25, (2) *FOXP3*/*CD8* ratio < 6.25.

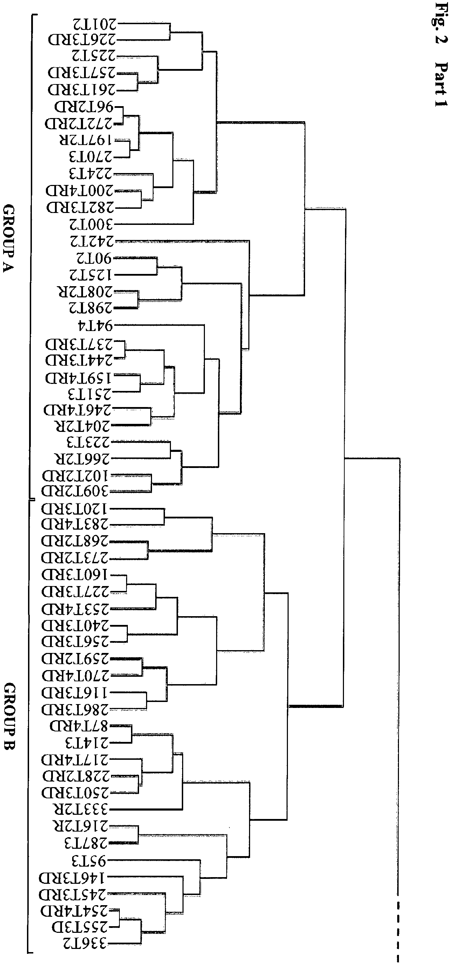

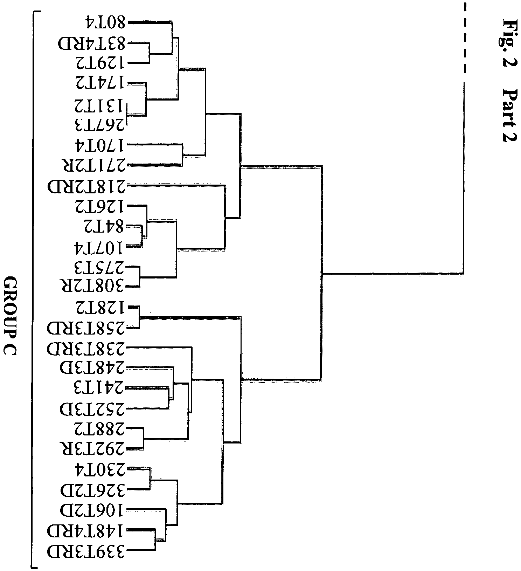

**Figure 2****.** Dendrogram of three MIBC tumor groups obtained from hierarchical cluster analysis of 83 MIBC tumors with the 3-gene signature comprising OX40L, CD8 and TIGIT. Group A: normal expression of each of OX40L, CD8 and TIGIT; Group B: over-expression of OX40L, and normal expression of each of CD8 and TIGIT; Group C: over-expression of each of OX40L, CD8 and TIGIT.

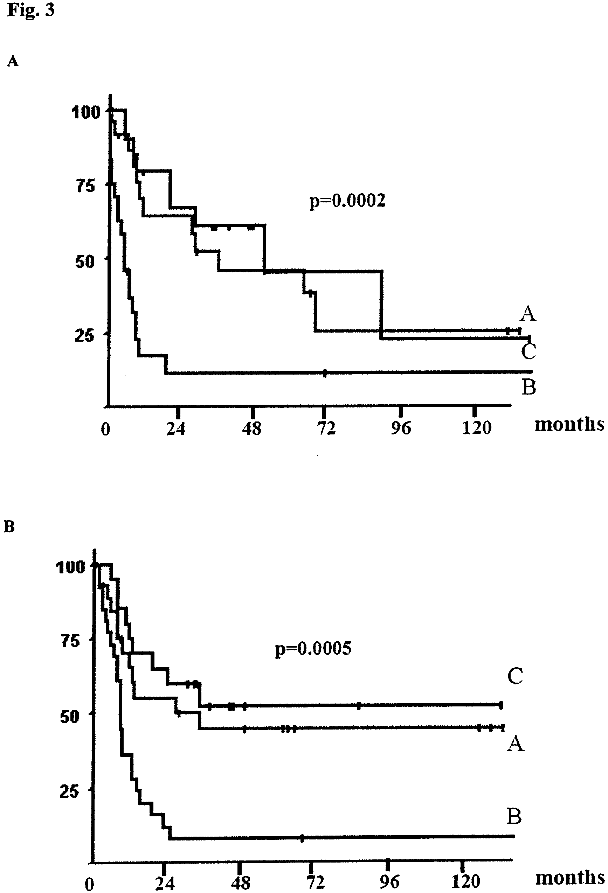

**Figure 3****. (A)** Kaplan-Meier curve comparing recurrence-free survival (RFS) of the three MIBC tumor Groups A to C identified in the analysis of 83 MIBC tumors with the 3-gene signature comprising OX40L, CD8 and TIGIT (p value is calculated using a log rank test). Group A: normal expression of each of OX40L, CD8 and TIGIT; Group B: over-expression of OX40L, and normal expression of each of CD8 and TIGIT; Group C: over-expression of each of OX40L, CD8 and TIGIT. **(B)** Kaplan-Meier curve comparing overall survival (OS) of the three MIBC tumor Groups A to C identified in the analysis of 83 MIBC tumors with the 3-gene signature comprising OX40L, CD8 and TIGIT (p value is calculated using a log rank test).

### Examples

### 1. MATERIALS AND METHODS

### 1.1. Patients and Samples

154 urothelial carcinoma samples from patients who had undergone transurethral bladder resection or radical cystectomy in our hospital between January 2002 and January 2006 were analyzed. Specimens of normal bladder tissue from 15 patients undergoing surgery unrelated to bladder tumors (transurethral resection of the prostate or prostatic adenomectomy) were used as sources of normal bladder tissues. All patients signed an informed consent. This study received approval from an institutional review board and was conducted according to the principles outlined in the Declaration of Helsinki.

Immediately after surgery, tumor samples from each patient were frozen in liquid nitrogen and stored at -80°C (for RNA extraction) and fixed in formaldehyde. Each tumor was reviewed by two pathologists (DD and MS) blinded to the clinical outcomes. Tumors were re-staged according to the 2009 American Joint Committee on TNM classification of bladder tumors and were graded according to OMS 2004 tumor-grading scheme as described in Sobin L et al., TNM classification of malignant tumors, UICC Int. Union Cancer. 2009. 262-5p; and Molinié V., Classification of bladder tumors, Prog Urol FMC 2006; 16: 7-10.

There were 25 women and 129 men with a median age of 70 years (range 31-91 years). Pathological staging showed NMIBC in 71 patients (25 low grade pTa, 17 high grade pTa, 29 high grade pT1) and high-grade MIBC in 83 patients. Standard follow-up visits were done according to current guidelines. Data were obtained from the patients' medical records. Clinical and histological parameters and survival characteristics of the two populations are presented in **Tables 3** (NMIBC) **and 4** (MIBC). Both populations characteristics are consistent with urothelial carcinoma presentation and evolution.

**Table 3: Clinical and pathological characteristics of the 71 members of the NMIBC group**

| | **No recurrence (n=25)** | **Recurrence (n=36)** | | **Muscle-invasive progression (n=10)** | |
|---|---|---|---|---|---|
| | **Number (%)** | **Number (%)** | **p-value*** | **Number (%)** | **p-value**** |
| **Age** (years) | | | | | |
| ≥60 | 19 (76.0) | 27 (75.0) | 0.93 | 10 (100.0) | 0.08 |
| <60 | 6 (24.0) | 9 (25.0) | | 0 (0.0) | |
| **Sex** | | | | | |
| Male | 22 (88.0) | 32 (88.9) | 0.91 | 9 (90.0) | 0.89 |
| Female | 3 (12.0) | 4 (11.1) | | 1 (10.0) | |
| **Smoking status** | | | | | |
| Non-smoker | 13 (52.0) | 15 (41.7) | 0.43 | 5 (50.0) | 0.81 |
| Smoker | 12 (48.0) | 21 (58.3) | | 5 (50.0) | |
| **History of NMIBC** | | | | | |
| No | 22 (88.0) | 13 (36.1) | **<0.0001** | 4 (40.0) | 0.31 |
| Yes | 3 (12.0) | 23 (63.9) | | 6 (60.0) | |
| **Associated pTis** | | | | | |
| No | 25 (100.0) | 36 (100.0) | 0.99 | 8 (80.0) | **0.0004** |
| Yes | 0.0 (0.0) | 0 (0.0) | | 2 (20.0) | |
| **Grade** | | | | | |
| Low grade | 10 (40.0) | 14 (38.9) | 0.93 | 1 (10.0) | 0.07 |
| High grade | 15 (60.0) | 22 (61.1) | | 9 (90.0) | |
| **Tumor stage** | | | | | |
| Ta | 15 (60.0) | 24 (66.7) | 0.59 | 3 (30.0) | **0.043** |
| T1 | 10 (40.0) | 12 (33.3) | | 7 (70.0) | |

| | | | | | |
|---|---|---|---|---|---|
| * Chi² test (recurrence versus no recurrence) ** Chi² test (muscle-invasive progression versus others) | | | | | |

For NMIBC, the median follow-up was 57.4 months (range 1-158) (mean follow-up, 61 months). Among the 71 cases of NMIBC, 36 (50.7%) recurred as NMIBC during the follow-up. The progression rate to a muscle-invasive tumor was 14.1% (10 patients).

**Table 4: Clinical and pathological characteristics of the 83 members of the MIBC group**

| | | **Disease-free survival (n=48)** | | **Overall survival (n=46)** | |
|---|---|---|---|---|---|
| | **Number of patients (%)** | **Number of events (%)^{a}** | **p-value*** | **Number of events (%)^{b}** | **p-value*** |
| **Age** (years) | | | | | |
| ≥60 | 61 (73.5) | 40 (65.5) | **0.017** | 39 (63.9) | **0.009** |
| <60 | 22 (26.5) | 8 (36.4) | | 7 (31.8) | |
| **Sex** | | | | | |
| Male | 66 (79.5) | 36 (54.5) | 0.23 | 38 (57.6) | 0.44 |
| Female | 17 (20.5) | 12 (70.6) | | 8 (47.1) | |
| **Smoking status** | | | | | |
| Non-smoker | 34 (41.0) | 18 (52.9) | 0.45 | 12 (35.3) | **0.002** |
| Smoker | 49 (59.0) | 30 (61.2) | | 34 (69.4) | |
| **History of NMIBC** | | | | | |
| No | 59(71.1) | 30 (50.8) | **0.043** | 31 (52.5) | 0.41 |
| Yes | 24 (28.9) | 18 (75.0) | | 15 (62.5) | |
| **Associated pTis** | | | | | |
| No | 73 (88.0) | 43 (58.9) | 0.59 | 40 (54.8) | 0.76 |
| Yes | 10 (12.0) | 5 (50.0) | | 6 (60.0) | |
| **Tumor stage** | | | | | |
| T2 | 34 (41.0) | 17(50.0) | 0.10 | 13 (38.2) | **0.009** |
| ≥T3 | 49 (59.0) | 31 (63.3) | | 33 (67.3) | |
| **Lymph node status** | | | | | |
| N- | 58 (69.9) | 27 (46.6) | **0.002** | 25 (43.1) | **0.0006** |
| N+ | 25 (30.1) | 21 (84.0) | | 21 (84.0) | |

| | | | | | |
|---|---|---|---|---|---|
| ^{a} first recurrence (local or metastatic) ; ^{b} death * Chi² test | | | | | |

For MIBC, the median follow-up was 12,3 months (range 1-152 months) (mean follow-up, 28 months). During the follow-up period, fourty-one patients (48.8%) died of bladder cancer and five patients (5.9%) died from unrelated causes.

### 1.2. Gene selection

Based on literature on antitumor immunity, the thirty-three genes identified in **Table 5,** which are known to be involved in immune process, including PD-1, CTLA4, IDO and TIGIT pathways, tumor necrosis factor receptor superfamily members (and especially *OX40* and its ligand *OX40L*), and other specific cellular markers of immune response as *CD4, CD8, INFG, TGFB3* and *IL10* were selected.

**Table 5: Selected Genes**

| **Gene** | **Accession number** | **Gene definition / encoded protein** |
|---|---|---|
| CD8 | NM_001768.6 | CD8a molecule |
| CD4 | NM_000616.4 | CD4 molecule |
| FOXP3 | NM_014009.3 | forkhead box P3 |
| PRF1 | NM_001083116.1 | Perforine |
| GZMA | NM_006144.3 | Granzyme A |
| INFG | NM_000619.2 | interferon, gamma |
| IL10 | NM_000572.2 | interleukin 10 |
| TGFB3 | NM_003239.3 | transforming growth factor beta 3 |
| TIGIT | NM_173799.3 | T cell immunoreceptor with Ig and ITIM domains |
| PVR | NM_006505.4 | Poliovirus receptor |
| CD96 | NM_198196.2 | CD96 molecule |
| CD226 | NM_006566.3 | CD226 molecule |
| OX40 | NM_003327.3 | tumor necrosis factor receptor superfamily, member 4 |
| OX40L | NM_003326.4 | tumor necrosis factor (ligand) superfamily, member 4 |
| TNFSF9 | NM_003811.3 | tumor necrosis factor (ligand) superfamily, member 9 |
| TNFRSF9 | NM_001561.5 | tumor necrosis factor receptor superfamily, member 9 |
| TNFSF18 | NM_005092.3 | tumor necrosis factor (ligand) superfamily, member 18 |
| TNFRSF18 | NM_004195.2 | tumor necrosis factor receptor superfamily, member 18 |
| IDO1 | NM_002164.5 | indoleamine 2,3-dioxygenase 1 |
| IDO2 | NM_194294.2 | indoleamine 2,3-dioxygenase 2 |
| TDO2 | NM_005651.3 | Tryptophan 2,3-dioxygenase |
| LGALS9 | NM_009587.2 | lectin, galactoside-binding, soluble, 9 |
| TIM3 | NM_032782.4 | hepatitis A virus cellular receptor 2 |
| LAG3 | NM_002286.5 | lymphocyte-activation gene 3 |
| ICOSLG | NM_015259.5 | inducible T-cell co-stimulator ligand |
| ICOS | NM_012092.3 | inducible T-cell co-stimulator |
| PD-L1 | NM_014143.3 | CD274 molecule |
| PD-L2 | NM_025239.3 | Programmed cell death 1 ligand 2 |
| PD-1 | NM_005018.2 | Programmed cell death 1 |
| CTLA4 | NM_005214.4 | Cytotoxic T-lymphocyte-associated protein 4 |
| CD28 | NM_006139.3 | CD28 molecule |
| CD80 | NM_005191.3 | CD80 molecule |
| CD86 | NM_175862.4 | CD86 molecule |

One endogenous RNA control gene was chosen, namely *TBP* (Gen-Bank Accession No. NM_003194) which encodes the TATA box-binding protein.

### 1.3. Quantitative real-time polymerase chain reaction (RT-PCR)

RT-PCR was carried out according to the procedure described in Pignot et al., Large-scale real-time reverse transcription-PCR approach of angiogenic pathways in human transitional cell carcinoma of the bladder: identification of VEGFA as a major independent prognostic marker, Eur Urol 2009; 56: 678-88.

Quantitative values were obtained from the cycle threshold (Ct) number at which the increase in the signal associated with exponential growth of PCR products was first detected. Each sample was normalized based on its *TBP* content. Results, expressed as N-fold differences in target gene expression relative to the *TBP* gene, and termed "N*target*"*,* were determined as N*target* = 2^{ΔCtsample}, where the ΔCt value of the sample was determined by subtracting the Ct value of the target gene from the Ct value of the *TBP* gene. N*target* values of the samples were subsequently normalized such that the median of the 15 normal bladder Ntarget values was 1. For 2 genes, namely TNFRSF18 and IDO2, because of a low level of expression, the mRNA values of the samples were normalized such that a Ct value of 35 was 1.

For each investigated gene, mRNA values of≥3 or more were considered to represent marked overexpression. Conversely, mRNA values of ≤0.33 were considered to represent marked under-expression.

### 1.4. CD8 and FOXP3 protein expression

Representative blocks of paraffin-embedded tumor were avalaible for 108 patients (50 NMIBC and 58 MIBC). For each tumor, two observers, including at least one expert pathologist, selected the tumor block containing the highest density of immune cells on hematoxylin and eosin-safran-stained slides. Briefly, serial 5 µm tissue sections were deparaffinized, rehydrated and pretreated in appropriate buffer for antigen retrieval in Leica automat. Tissue slides were then incubated at 48°C with a primary antibody [anti-CD8 (SP16, Spring-bioscience) or anti-FOXP3 (263A/E7, Abcam) at a concentration of 1:100], followed by incubation with appropriated secondary antibodies.

### 1.5. Scoring of protein expression

We performed a semi quantitative analysis with a score of 0 (no positive cells), 1+ (few positive cells) and 2+ (numerous positive immune cells). The same score was applied to CD8 and FOXP3 on tumor immune infiltrating cells. All quantification was done blindly by an expert pathologist (D.D.).

### 1.6. Statistical analyses

The clinicopathological features of NMIBC and MIBC were tested for their association with tumor recurrence and survival, using the Student's t-test for continuous variables or, the chi-squared test for qualitative variables.

The distribution of mRNA levels was analyzed using median values and ranges. Relationships between mRNA levels of the different target genes and clinical and histological parameters were tested using two non-parametric tests: the Mann-Whitney U-test and the Kruskal-Wallis H-test (a link between one qualitative parameter and one quantitative parameter).

Overall survival (OS) was calculated from the date of surgery until death or the last follow-up. Disease-free survival (DFS) was defined as the time elapsed from the date of surgery until the first local relapse or first metastasis. For NMIBC, progression-free survival (PFS) was defined as the time elapsed from the date of surgery until progression to muscle-invasive disease. Patients were censored if they had not experienced the end-point of interest at the time of last follow up. Survival curves were derived from Kaplan-Meier estimates. The log-rank test was used to compare survival distributions between subgroups. Hazard ratios (HR) associated with OS or DFS were calculated with their 95% confidence interval for each covariate from Cox proportional hazards regression model. The prognostic impact of mRNA levels, adjusted for the other prognostic factors, was assessed in multivariate analyses using the Cox's proportional hazards regression model.

Differences between two populations were judged significant at confidence levels greater than 95% (p<0.05).

### Example 1: Determination and analysis of mRNA expression of the 33 selected genes

The mRNA expression levels of the 33 selected genes were determined by RT-PCR in normal tissue bladder samples and the tumor bladder samples of the cohorts of NMIBC and MIBC patients.

All 33 selected genes, except *LGALS9,* showed a significantly different expression profile in MIBC compared with NMIBC. All differently expressed genes were over-expressed in MIBC compared with NMIBC, except CD96.

With respect to NMIBC, it was found that 15 of the 33 genes (45.5%) were significantly deregulated (differentially expressed) in NMIBC compared to normal bladder tissue (p<0.05); 8 were under-expressed: *PD-L2, CD4, CD8, CD226, TIM3, IFNG, IL10*, and *TGFB3*; and 7 were overexpressed: *CD80, LGALS9, CD96, TNFRSF9, TDO2, OX40* and *FOXP3.* Further, 15 genes showed significant differences in levels of expression according to the stage of the tumor in the NMIBC patients, the majority of them being over-expressed in pT1 tumors compared with pTa tumors. On the other hand, only 2 genes, namely *TNFSF9* and *CD86,* showed different expression profiles according to the grade of the tumor - low grade versus high grade.

In the MIBC tumor samples, 24 of the 33 genes (72.7%) were significantly deregulated in the MIBC samples compared with normal bladder tissue samples (p<0.05). All 24 differentially expressed genes showed over-expression, except *TGFB3* which was significantly under-expressed.

Clustering analyses of the 33 selected genes showed a high concordance between expression levels of all these genes, especially between epithelial genes on the one hand and stromal genes on the other hand.

### Example 2: Correlation between mRNA expression levels in the NMIBC group and prognosis

### 2.1. Univariate analysis

In the NMIBC group, among the 33 studied genes, the expression values of *FOXP3* and *OX40L* were associated with recurrence-free survival, whereas only *FOXP3* expression values were associated with progression-free survival, in univariate analyses. Kaplan-Meier survival analyses revealed that over-expression (>3-fold) of *OX40L* was significantly associated with a higher risk of recurrence (log rank test: p=0.0023). *FOXP3* overexpression (>3-fold) was significantly associated with a higher risk of both recurrence and progression to muscle-invasive tumors (p=0.0062 and p=0.018, respectively). As described in several previous studies, FOXP3 and CD8 expression were co-analysed, by defining *FOXP3*/*CD8* ratio. *FOXP3*/*CD8* ratio was determined using *FOXP3* and *CD8* mRNA value on each NMIBC sample in order to obtain a quantitative value (median value [range] = 6.25 [0.94-45.01]). Tumors with a high *FOXP3*/*CD8* ratio ≥ 6.25) had a significant worse prognosis in terms of recurrence-free survival than those with a lower ratio (p=0.010); see **Fig. 1****.**

### 2.2. Multivariate analysis

Multivariate analysis using the Cox proportional hazards model was carried out to assess the relative influence of clinicopathological and molecular prognostic factors significantly associated with outcome of NMIBC, i.e. history of NMIBC, *FOXP3*/*CD8* ratio and *OX40L* status, for recurrence, and stage, grade and *FOXP3* status, for progression to muscle-invasive tumor, at a significance level of 5% in univariate analyses. The results are summarized in **Table 6.**

**Table 6: Factors affecting disease-free survival of NMIBC in multivariate analysis.**

| **Prognostic factor** | **Disease-free survival** | | |
|---|---|---|---|
| | Adjusted HR | 95% CI | p * |
| **History of NMIBC** | 2.66 | [1.43-4.94] | **0.0019** |
| **FOXP3/CD8 *ratio*** | 2.11 | [1.15-3.88] | **0.016** |
| **OX40L *overexpression*** | 2.27 | [1.04-4.97] | **0.0039** |

| | | | |
|---|---|---|---|
| * Cox model HR: hazard ratio; CI: confidence interval | | | |

As can be seen, *FOXP3*/*CD8* ratio and *OX40L* status are prognostic factors that are significantly associated with disease-free survival (p=0.016 and p=0.0039, respectively), whereas none of them was associated with progression-free survival.

### Example 3: Correlation between mRNA expression levels in the MIBC group and prognosis

### 3.1. Univariate analysis

In the MIBC group, among the 33 studied genes, over-expression (>3-fold) of *OX40L* was significantly associated with a poorer prognosis in terms of both RFS and OS (p=0.027 and p=0.014, respectively). On the other hand, over-expression (>3-fold) of *CD8* was significantly associated with a better prognosis in terms of both RFS and OS (p=0.024 and p=0.029, respectively). The expression values of *TIGIT* were also associated with overall survival (p=0.042).

### 3.2. Identification of a molecular signature predictive of MIBC prognosis

The unsupervised hierarchical clustering analyses of 83 MIBC tumors using the 3-gene signature comprising *OX40L, CD8* and *TIGIT,* which was previously identified as associated with survival outcomes in MIBC, revealed three major clusters composed of 29 (Group A), 27 (Group B) and 27 (Group C) members, respectively; see **Fig. 2****.** Regarding the mRNA expression profile, Group A was characterized by normal expression of the genes of the 3-gene signature - *OX40L, CD8* and *TIGIT-* in MIBC tumor sample tissue compared to normal tissue samples. Group B was characterized by a marked over-expression of *OX40L,* and Group C was characterized by a simultaneous over-expression of each of *OX40L, CD8* and *TIGIT,* see **Table 7.**

**Table 7: mRNA median value [range] and over-expression rates of OX40L, CD8 and TIGIT in the three tumor groups (bold value for overexpressed genes (median ≥ 3-fold), p value is calculated using Kruskal Wallis H-test).**

| **Gene** | **Group A (n=29)** | **Group B (n=27)** | **Group C (n=27)** | **p** |
|---|---|---|---|---|
| ***CD8* mRNA value** | 0.65 [0.09-2.30] | 0.66 [0.20-2.53] | **4.70 [2.29-45.55]** | **<0.0001** |
| **Over-expression (%)** | 0 (0.0) | 0 (0.0) | **23 (85.2)** | |
| ***OX40L* mRNA value** | 1.35 [0.00-14.58] | **20.09 [9.32-110.27]** | **11.79 [2.53-86.74]** | **<0**.**0001** |
| **Over-expression (%)** | 10(34.5) | **27 (100.0)** | **26 (96.3)** | |
| ***TIGIT* mRNA value** | 2.33 [0.26-8.44] | 2.48 [0.40-12.53] | **10.53 [5.56-68.06]** | **<0.0001** |
| **Over-expression (%)** | 9(31.0) | 8 (29.6) | **27 (100.0)** | |

No significant differences between the three groups were observed with regard to clinicopathological factors. However, survival analyses comparing these three MIBC tumor groups showed significant differences in terms of both RFS and OS (log rank test: p=0.0002, and p=0.0005, respectively). The Kaplan-Meier survival curves showing the poorer outcome associated with cluster Group B are presented in **Fig**. **3A** (RFS) and **Fig**. **3B** (OS), respectively. Five-year disease-free and overall survival rates were 11.2% and 8.3% for patients in Group B versus 47.9% and 44.2% for patients in Group A and 47.1% and 52.8% for patients in Group C, respectively.

### 3.3. Multivariate analysis

Multivariate analysis using the Cox proportional hazards model was carried out to assess the relative influence of clinicopathological and molecular prognostic factors significantly associated with MIBC outcome, i.e. tumor stage, lymph node status and the 3-gene signature, at a significance level of 5% in univariate analyses. The results are summarized in **Table 8.**

**Table 8: Factors affecting overall and disease-free survival of MIBC in multivariate analysis.**

| **Prognostic factor** | **Disease-free survival (DFS)** | | | **Overall survival (OS)** | | |
|---|---|---|---|---|---|---|
| | Adjusted HR | 95% CI | p * | Adjusted HR | 95% CI | p * |
| | | | 0.19 | | | 0.23 |
| **T Stage** | | | | | | |
| ≤T2 | 1 | | | 1 | | |
| T3-4 | 1.62 | [0.79-3.34] | | 1.57 | [0.75-3.28] | |
| **N+ status** | 3.37 | [1.56-T.30] | **0.002** | 3.26 | [1.61-6.60] | **0.001** |
| **3-gene signature** | | | **0.0007** | | | **0.007** |
| Group C | 1 | | | 1 | | |
| Group A | 2.08 | [1.36-3.19] | | 1.74 | [1.16-2.61] | |
| Group B | 4.34 | [1.85-10.19] | | 3.04 | [1.36-6.80] | |

| | | | | | | |
|---|---|---|---|---|---|---|
| * Cox model HR: hazard ratio ; CI: confidence interval | | | | | | |

As can be seen, lymph node status and the 3-gene signature are prognostic factors that are significantly associated with both disease-free survival (p=0.002 and p=0.0007, respectively) and overall survival (p=0.001 and p=0.007, respectively).

### Example 4: Immunohistochemistry analysis

Table 9 shows the results of CD8 and FOXP3 staining on immune cells. Protein expression (≥1+) of CD8 on immune cells was significantly more common in MIBC compared with NMIBC (p=0.0084). Importantly, we found a strong correlation between RT-PCR analysis and immunohistochemistry analysis, and thus a high correlation between mRNA and protein expression for both CD8 and FOXP3 (p=0.00000016 and p=0.00000093 respectively).

**Table 9: Immunohistochemistry results for CD8 and FOXP3 on immune cells**

| | **CD8 (immune cells)** | | | | **FOXP3 (immune cells)** | | | |
|---|---|---|---|---|---|---|---|---|
| | **0+(%)** | **1+ (%)** | **2+ (%)** | **p*** | **0+ (%)** | **1+ (%)** | **2+ (%)** | **p*** |
| **All tumors (n=108)** | 19 (17.6) | 53 (49.1) | 36 (33.3) | | 26 (24.1) | 46 (42.6) | 36 (33.3) | |
| **NMIBC (n=50)** | 14 (28.0) | 27 (54.0) | 9 (18.0) | **0.0084** | 16 (32.0) | 21 (42:0) | 13 (26.0) | 0.074 |
| **MIBC (n=58)** | 5 (8.6) | 26 (44.8) | 27 (46.0) | | 10 (17.2) | 25 (43.1) | 23 (39.7) | |

| | | | | | | | | |
|---|---|---|---|---|---|---|---|---|
| *Chi-2 test (0+ versus 1+/2+) | | | | | | | | |

## Claims

1. A method of determining a prognosis of a recurrence-free survival in a bladder cancer patient comprising:
determining mRNA or protein expression levels for a gene panel in a tumor sample of a bladder cancer patient;
wherein the gene panel consists of CD8 (NM_001768.6, CD8a molecule), FOXP3 (NM_014009.3, forkhead box P3), TIGIT (NM_173799.3, T cell immunoreceptor with Ig and ITIM domains) and OX40L (NM_003326.4, tumor necrosis factor (ligand) superfamily, member 4);
wherein a bladder cancer patient having a high expression level for OX40L, and (i) a normal expression level for each of CD8 and TIGIT or (ii) a FOXP3/CD8 ratio of ≥ 6.25 (i.e. equal or more than 6.25), has a prognosis of a decreased probability of recurrence-free survival.

2. The method of claim 1, wherein the bladder cancer patient is a non-muscle invasive bladder cancer (NMIBC) patient.

3. The method of claim 1, wherein the bladder cancer patient is a muscle invasive bladder cancer (MIBC) patient.

4. The method of any one of claims 1 to 3, wherein the tumor sample is a paraffin-embedded biopsy sample.

5. The method of claim 2 or 4, comprising:
determining mRNA or protein expression levels for the gene panel in the tumor sample of the patient; wherein the gene panel consists of CD8, FOXP3 and OX40L;
wherein a NMIBC patient having a high expression level for OX40L, and a FOXP3/CD8 ratio of ≥ 6.25 has a prognosis of a decreased probability of recurrence-free survival.

6. The method of claim 3 or 4, comprising:
determining mRNA or protein expression levels for the gene panel in the tumor sample of the patient; wherein the gene panel consists of CD8, TIGIT and OX40L;
wherein a MIBC patient having a high expression level for OX40L and a normal expression level for each of CD8 and TIGIT has a prognosis of a decreased probability of recurrence-free survival.

7. A method of determining a prognosis of overall survival in a MIBC patient, wherein the method comprises:
determining mRNA or protein expression levels for a gene panel in a tumor sample of the patient; wherein the gene panel consists of CD8, TIGIT and OX40L;
wherein a MIBC patient having a high expression level for OX40L and a normal expression level for each of CD8 and TIGIT has a prognosis of a decreased probability of overall survival.

8. The method of any one of claims 1 to 7, wherein the mRNA expression profile of the genes of the gene panel is determined by an amplification- and/or hybridization-based assay.

9. The method of any one of claims 1 to 7, wherein the protein expression profile of the proteins encoded by the genes of the gene panel is determined by immunohistochemistry.

10. The method of any one of claims 1 to 9, further comprising, determining the level of one or more normalization control(s) in the sample.

11. The method of claim 10, wherein the normalization control is an endogenous RNA.

12. A kit for determining a MIBC or NMIBC patient's prognosis of recurrence-free or overall survival comprising means for determining the expression profile of CD8, FOXP3 and OX40L; or CD8, OX40L and TIGIT; or CD8, FOXP3, OX40L, and TIGIT; in a tumor sample from a patient.

13. Use of a mRNA or protein expression profile of CD8, FOXP3 and OX40L; or CD8, OX40L and TIGIT; or CD8, FOXP3, OX40L, and TIGIT; in a tumor sample from a NMIBC or MIBC patient for determining a MIBC or NMIBC patient's prognosis of recurrence-free or overall survival.

14. The use of a mRNA or protein expression profile according to claim 13, wherein over-expression of OX40L, and an expression ratio of FOXP3/CD8 of ≥ 6.25 in the tumor sample from a NMIBC patient is indicative for a higher risk of recurrence.

15. The use of a mRNA or protein expression profile according to claim 13, wherein over-expression of OX40L, and normal expression of each of CD8 and TIGIT in the tumor sample from a MIBC patient is indicative for a higher risk of recurrence and/or a relatively lower chance of survival.
